# EUROPEAN PATENT APPLICATION

(11) **EP 2 476 673 A1**
(43) Date of publication of application: **18.07.2012**
(21) Application number: 10197024.2
(22) Date of filing: 27.12.2010
(51) Int. Cl.: C07D 303/38

(54) **Radiation curable compositions and compounds**

(71) Applicant: Cytec Surface Specialties, S.A., 1070 Brussels (BE)
(72) Inventor: Salviato, Jean-Yves, 6210, Les Bons Villers (BE); Fischer, Thomas, 8111 Judendorf-Strassengel (AT); Burie, Peter, 9050 Gentbrugge (BE)
(74) Representative: Schoofs, Hilde

(57) **Abstract**

A compound is disclosed, which together with at least one epoxy function and at least one ethylenically unsaturated function, whereby the epoxy functions may be unreacted or having been reacted with a moiety reactive toward an epoxy function, also comprises at least one sulphur-containing anionic moiety. The compound is preferably soluble or dispersible in an aqueous medium. Further disclosed are radiation curable compositions, preferably aqueous, and functional compositions made with the compound, articles coated therewith, uses of the compound in coatings and inks, and processes for the production of the compound and its derivatives.

## Description

### FIELD OF THE INVENTION

The present invention relates to radiation curable compositions containing an ethylenically unsaturated compound containing unreacted and/or reacted epoxy functions, and which are advantageously water based. More particularly, the invention relates to the ethylenically unsaturated derivative containing the epoxy functions, and which may be dispersed or dissolved in an aqueous medium thanks to a hydrophilic functionality which is internal to the molecule.

### BACKGROUND OF THE INVENTION

The reaction between epoxy resins and acrylic acid is one the most economical synthetic routes leading to acrylate oligomers which may be cured through ultraviolet (UV) or electron beam (EB) radiation. The reaction products are commonly called 'epoxy acrylates'. They allow the user to obtain a fast cure under UV radiation, and they result in coatings having a good hardness, and good chemical and water resistance.

Epoxy acrylates however have two main limitations: their viscosity is high and the cured films suffer from a lack of flexibility. Decreasing the viscosity of an epoxy acrylate formulation may be achieved by including reactive diluents. These however have a negative impact on the characteristics of the films, primarily by making them more brittle. The viscosity of the formulation may also be reduced by including a solvent, but solvent-based consumer products have recently become less desired because of safety and industrial hygiene concerns.

Water-based epoxy acrylates have therefore been developed. These may permit to obtain formulations having a very low viscosity without the need to add any reactive monomers or solvent.

Anionic, cationic and non-ionic dispersions of epoxy acrylates in water have been described. Anionic dispersions have the advantage to be stable over a range of pH, typically ranging from neutral to moderately basic. They are suitable for a large variety of substrates and thanks to their pH stability they are also compatible with most of the common additives used in the coating industry, such as pigments and rheology modifiers. Many of these dispersions use emulsifiers or other surfactant ingredients in order to stabilise the dispersion. The drawback of such external dispersion stabilisers is that these may bleed out of the film made with the epoxy acrylate dispersion.

In order to overcome the problems of external dispersion stabilisers, epoxy acrylate systems have been developed wherein the resin is modified by building hydrophilic groups, such as carboxylic acid groups, into the resin molecules. These carboxylic acid groups are usually neutralized and present in the salt form, which improves the stability over a wide pH range.

A well-known method for obtaining an internal stabilized dispersion of epoxy acrylates is to react in a first step acrylic acid with an excess of epoxy resin and to react in a second step the remaining free glycidyl functions with an excess of a polyacid or another component which can generate at least one acid group. The remaining free acid is subsequently preferably neutralized, such as with a tertiary amine. Following references are non-exhaustive examples of water based epoxy acrylates obtained by this principle. In US 4,025,692, water based epoxy acrylates are obtained by reaction of a prepolymer of a bisphenol-type epoxy resin with (meth)acrylic acid, optionally further reacting the reaction product with phosphoric acid or maleic anhydride, followed by neutralisation with an amine. In CN 101037497, the resin is reacted with maleic anhydride, and in US 7,268,171 with diphosphorous pentaoxide.

Ionic water dispersible epoxy acrylate which contain both quaternary ammonium and phosphate anion groups may be obtained according to US 5,512,607 by the reaction of an epoxy resin with phosphoric acid or ester and with tertiary amine. US 4,444,923 discloses an ionic polymer obtained from an epoxy resin esterified with a polymer containing carboxylic acid groups, further free acid groups being neutralised with a tertiary amine.

US 2006/0264527 discloses radiation curable compounds obtained in 3 sequential steps. A polyepoxide (A) having at least two epoxy groups per molecule is first reacted with an unsaturated fatty acid (B). In a second step, the double bonds in the adduct (AB) of the first step are reacted with a carboxylic acid containing olefinically unsaturated monomer (C), and in the third step the graft copolymers (ABC) are reacted with the isocyanate groups of a compound (DE) which contains also radiation curable olefinically unsaturated groups. An aqueous dispersion is made after neutralization of the remaining free carboxylic acid with lithium hydroxide.

The properties of these dispersions known in the art, and of the films made therewith, are still open for further improvement. Important properties are the curing rate, the viscosity and the stability of the dispersion, and the hardness, chemical and stain resistance of the cured product.

The present invention aims to obviate or at least mitigate one or more of the above described problems and/or to provide improvements generally.

### SUMMARY OF THE INVENTION

According to the invention, there is provided an improved ethylenically unsaturated chemical compound containing unreacted and/or reacted epoxy functions, a radiation curable composition, a functional composition, an article at least for a part coated, and processes for the production of these products as defined in any of the accompanying claims.

The invention provides a compound comprising at least one epoxy function and at least one ethylenically unsaturated function, whereby the epoxy functions present in the compound are independently selected from the group consisting of unreacted epoxy functions and epoxy functions which have been reacted with a moiety reactive toward an epoxy function, the compound further comprising at least one sulphur-containing anionic moiety.

In particular the present invention provides an ethylenically unsaturated compound prepared from a polyepoxide resin which compound further comprises at least one sulphur-containing anionic moiety, and which compound is selected from the group consisting of in an aqueous medium soluble and dispersible compounds, and combinations thereof. Preferred are in aqueous medium dispersible compounds.

The sulphur-containing anionic moieties in particular are moieties which are capable of, or in the presence of other hydrophilic moieties as part of the same molecule, at least contribute to the capability of rendering the compound of the invention soluble or dispersible in water, either directly or after the reaction with a neutralizing agent to form a salt. Especially moieties which contain sulphonate salt groups were found highly suitable for these purposes.

The invention further provides, in another embodiment, a radiation curable composition comprising at least one compound according to the present invention. Preferred herein are radiation curable aqueous compositions, more in particular dispersions in water.

In another embodiment, the present invention provides a functional composition selected from a coating, an ink, a varnish or an adhesive composition, which functional composition comprises at least one compound according to the present invention or comprises a radiation curable composition according to the present invention, which preferably is a radiation curable aqueous composition, more preferably is a dispersion in water.

The applicants have found that the compound according to the present invention may be readily dissolved or dispersed in an aqueous medium, and this without needing external emulsifiers and/or dispersion stabilisers, or solvents. The drawbacks of such ingredients are thus avoided. The resulting composition according to the present invention brings the advantage of a low viscosity, even when it is further complemented with other ingredients to form a functional composition, an advantage which is highly appreciated when the composition is spread over a substrate in the form of a thin film. This advantage may be achieved even with compositions having a high solids content. The applicants have further found that the radiation curable compositions comprising the compound according to the present invention bring the advantage of fast cure when compared to other similar curable systems. This relatively high curing rate allows for high processing speeds where the compositions according to the present invention are applied, and makes these compositions particularly suitable for in-the-field applications, such as coating treatments of floors and rooftops, in particular when located outdoors.

The applicants have further found that cured films produced with the compound according to the present invention may bring advantages of relatively high hardness, high gloss, high chemical resistance and high stain resistance, when compared to other curable systems also having internal emulsifiers. In particular chemical resistance and/or stain resistance are surprisingly improved. The applicants have further found that systems comprising the compound according to the present invention may be used with a wide variety of substrates, including wood, paper, plastics, glass, metal, ceramics and concrete. In particular, it has been found that these films show an excellent adhesion to metal, and this without the need for a primer and/or an adhesion promoter.

In yet another embodiment, the present invention provides an article which at least for a part is covered by a functional composition according to the present invention. The article preferably comprises at least a part made of a material selected from wood, plastic, glass, paper, metal, ceramics and concrete. The article is preferably selected from the group consisting of a tile, a wooden floor element, parquet, parquetry and furniture. The applicants have found that the present invention is very suitable for providing clear coat coatings, such as clear varnishes, which may be used for protecting wood and improving the aesthetic aspects thereof.

In yet another embodiment, the present invention provides a process for the production of the compound according to the present invention, comprising the steps of
(a) in a first step, producing a compound AB by reacting at least one compound B comprising in the same molecule at least one sulphur-containing anionic moiety and at least one moiety reactive toward an epoxy function, with at least one epoxy compound A, which comprises at least two epoxy functions, and, if compound B comprises per molecule at least two moieties reactive towards an epoxy function, optionally in the presence of at least one mono epoxy compound, whereby the unreacted epoxy functions are present in a stoichiometric excess relative to the number of moieties reactive toward an epoxy function, and
(b) in a second step, producing a compound ABC by reacting at least partially and preferably completely the residual free or unreacted epoxy functions in the product AB of step (a) with a compound C comprising at least one carboxylic acid moiety and at least one ethylenically unsaturated carbon-carbon double bond to form the compound according to the present invention.

In a further embodiment, the present invention provides the use of the compound according to the present invention for the at least partial covering of a substrate which preferably is selected from the group consisting of wood, paper, glass, metal, ceramics, concrete and plastics. The compound according to the present invention may also be used in a radiation curable printing ink, varnish or adhesive, including water based radiation curable inks, varnishes and adhesives.

The applicants have found that the compositions according to the present invention are suitable for use with a wide variety of substrates, and in a wide variety of functional compositions.

### DETAILED DESCRIPTION

In the context of the present invention, a moiety reactive toward an epoxy function is any chemical group or function containing ionizable hydrogen known in the art as being reactive to epoxy groups. Preferably the moiety is selected from the non-exhaustive group consisting of a hydroxyl function, an acid function, such as a carboxylic acid function, an anhydride function, an ethylenically unsaturated function such as a vinyl function, in particular a terminal vinyl function, a lactone function, a phosphonic ester function, a phosphoric ester function, an amide function, a cyanamide function, an oxazolidone function, a primary amine and a secondary amine function, more preferably selected from the group consisting of a hydroxyl function, an organic acid function, a primary amine and a secondary amine function.

Provided by the invention as one embodiment is an ethylenically unsaturated compound prepared from a polyepoxide resin which compound further comprises at least one sulphur-containing anionic moiety, and which compound advantageously is selected from the groups consisting of in an aqueous medium soluble compounds, and combinations thereof. Preferred are in aqueous medium dispersible compounds.

A polyepoxide or a polyepoxy compound is defined herein as a molecule or compound comprising at least two free or unreacted epoxy functions, also called oxirane functions, i.e. cyclic ether groups having two fully saturated carbon atoms. The polyepoxide may contain further epoxy functions which are reacted with a moiety reactive to an epoxy function. Preferably at least one of the epoxy functions is, or is part of, a glycidyl group, and more preferably all epoxy functions in the polyepoxide are, or are part of, a glycidyl group.

Typically the compound according to the present invention may be a monomer, an oligomer or a polymer. The ethylenically unsaturated epoxy compound of the invention preferably is an oligomer or a polymer molecule.

A dispersible compound according to the present invention is preferably a compound which, when mixed with water or an aqueous medium, is able to form a two-phase system of particles in water. The particles formed typically have an average particle diameter of at most 600 nm, often at most 300 nm and preferably at most 150 nm, and optionally at least 50 nm and preferably at least 80 nm.

A compound according to the present invention which is soluble in water or in an aqueous medium is preferably a compound which is able to form a homogeneous, single phase mixture when mixed with water over a concentration range of 5 to 75%wt of water, based on the total composition.

In the context of the present invention, an ethylenically unsaturated function is meant to designate a carbon-carbon double bond which under the influence of irradiation and/or a (photo)initiator may undergo free radical polymerization. The polymerisable ethylenically unsaturated groups are generally chosen from (meth)acrylic groups, preferably acrylic groups. In the present invention, the term "(meth)acryl' is to be understood as to encompass both acryl and methacryl compounds or derivatives, as well as mixtures thereof.

In an embodiment, the sulphur-containing anionic moiety of the compound according to the present invention is selected from a sulphur-containing acid moiety and a salt thereof. Preferably the sulphur-containing anionic moieties are selected from anionic moieties which comprise sulphonic acid or the salt thereof and/or from moieties which comprise sulphinic acid or the salt thereof. Particularly preferred are anionic moieties which comprise sulphonic acid or the salt thereof, with anionic moieties comprising sulphonate groups being most preferred.

In the embodiment with a sulphur-containing acid moiety, the applicants prefer the salt form over the acid form, because they have found that the salt form is more stable, more readily survives the chemical reactions occurring as part of the process of the present invention, provides more stable dispersions, and brings a lower risk for developing colour during production or storage. With more of the sulphur-containing anionic moieties surviving, the aqueous compositions have a higher stability over time and under a wider range of conditions, such as temperature and pH. The salt may be obtained by neutralising an acid anionic moiety with a monobasic material such as an alkali metal hydroxide, a tertiary monoamine or ammonium hydroxide, The salt of an alkali metal is preferred, because it avoids the possible odour, toxicity and industrial hygiene problems associated with amino compounds, and most preferred is the sodium salt.

In one embodiment of the present invention wherein the compound is a salt, the cation selected for the salt is a non-volatile cation, Suitable cations may for instance be selected from the alkali metals, most preferably sodium or potassium. The applicants have found that in this embodiment the dispersions may be odourless, and may be made to comply with the requirements of a "zero volatile organic compounds" or "zero VOC" products, or at least approach these requirements fairly closely. This brings significant advantages in terms of industrial hygiene and public acceptance.

In a preferred embodiment of the present invention, the sulphur-containing anionic moiety is a sulphonic-acid-containing moiety, preferably a moiety comprising a salt of sulphonic acid, more preferably the salt being selected from a sodium, a potassium and an ammonium salt, and mixtures thereof, even more preferably the sodium salt.

In an embodiment, the applicants prefer to have from 1 to 3 sulphur-containing anionic moieties per molecule of compound according to the present invention, preferably 1 to 3 sulphonic acid or sulphonate salt groups per molecule. The applicants prefer that the total of sulphur-containing anionic moieties content, more in particular the total of sulphonic acid and/or sulphonate group content, is in the range of from 0.1 to 1 equivalents per kg of the compound, preferably of from 0.25 to 0.5 equivalents per kg, more preferably from 0.30 to 0.35 equivalents per kg. In the context of this invention, an equivalent is defined as the amount of a substance which will react with one gmole of monofunctional reactant, such as one mole of hydrogen ions (H+) in an acid-base reaction.

In an embodiment of the compound according to the present invention, other hydrophilic moieties, such as non-ionic or anionic or a combination thereof, may be incorporated into the compound if desired. Anionic moieties comprising carboxylic or phosphonate acid or the salt thereof, when incorporated, typically are limited in content to at most 50% of the total acid or anion equivalents. Non-ionic moieties, when incorporated, typically are limited to 20% of the total solids weight of the compound.

In a preferred embodiment, the compound of the present invention is a (meth)acrylated ethylenically unsaturated epoxy compound. Acrylic acid, and to a lesser extent also methacrylic acid, provides one of the most economical routes for synthesizing an epoxy acrylate oligomer suitable for radiation curable compositions.

The compound according to the present invention may be prepared in various ways but preferably is prepared following a process comprising the steps of
(a) in a first step, producing a compound AB by reacting at least one compound B comprising in the same molecule at least one sulphur-containing anionic moiety and at least one moiety reactive toward an epoxy function, with at least one epoxy compound A, which comprises at least two epoxy functions, and, if compound B comprises at least two moieties reactive towards an epoxy function, optionally in the presence of at least one mono epoxy compound, whereby the unreacted epoxy functions are present in a stoichiometric excess relative to the number of moieties reactive toward an epoxy function, and
(b) in a second step, producing a compound ABC by reacting at least partially and preferably completely the residual free or unreacted epoxy functions in the product AB of step (a) with a compound C comprising at least one carboxylic acid moiety and at least one ethylenically unsaturated carbon-carbon double bond to form the compound according to the present invention.

If desired, the compound of the present invention, in any of the embodiments thereof described herein, may be chain extended. Accordingly the present invention also relates to a process which comprises the steps of:
(a) in a first step, producing a compound AB by reacting at least one compound B comprising in the same molecule at least one sulphur-containing anionic moiety and at least one moiety reactive toward an epoxy function, with at least one epoxy compound A, which comprises at least two epoxy functions, and, if compound B comprises at least two moieties reactive towards an epoxy function, optionally in the presence of at least one mono epoxy compound, whereby the unreacted epoxy functions are present in a stoichiometric excess relative to the number of moieties reactive toward an epoxy function,
(b) in a second step, producing a compound ABC by reacting at least partially and preferably completely the residual free or unreacted epoxy functions in the product AB of step (a) with a compound C comprising at least one carboxylic acid moiety and at least one ethylenically unsaturated carbon-carbon double bond, and
(c) optionally, in a further step producing a chain extended compound ABCD by reacting the product ABC of step (b) with at least one chain extender D containing groups capable of reacting with residual free or unreacted epoxy functions and/or capable of reacting with (meth)acrylic double bonds via a Michael addition reaction to provide chain extension, to form the chain extended compound according to the present invention.

The present invention further relates to ethylenically unsaturated epoxy compounds obtained according to these processes.

Compounds A may be polyepoxy compounds, and typically are polyglycidyl compounds. A polyglycidyl compound is meant to designate a compound comprising at least two unreacted glycidyl functions. A polyglycidyl compound may comprise further free or unreacted glycidyl functions as well as glycidyl functions which have reacted with a moiety reactive to an epoxy function.

Suitable as compound A are aromatic and/or aliphatic polyglycidyl compounds, in which diglycidyl compounds are preferred.

Aromatic diglycidyl compounds derived from Bisphenol A and Bisphenol F are preferred as compound A. Particularly preferred are Bisphenol A diglycidyl ether, Bisphenol F diglycidyl ether and their ethoxylated and/or propoxylated equivalents. It is also possible to employ diglycidyl esters, such as diglycidyl phthalate, N,N-diglycidyl aniline, N,N-diglycidyl-4-glycidyloxyaniline, diglycidyl hexahydrophthalate.

Aliphatic diglycidyl compounds may also be used, such as those derived from alpha, omega diols having 4 to 12 carbon atoms or from polyoxyalkylenediols, especially polyethylene glycol, polypropylene glycol or mixtures thereof which contain oxyalkylene groups, for example 1,4-butanediol diglycidyl ether, 1,6-hexanediol diglycidyl ether, cyclohexane-dimethanol diglycidyl ether, polyethylene glycol diglycidyl ether, polypropylene glycol diglycidyl ether, hydrogenated Bisphenol A diglycidyl ether, hydrogenated Bisphenol F diglycidyl ether, and their ethoxylated and/or propoxylated equivalents. It is also possible to employ diglycidyl esters. Preferred aliphatic diglicydyl compounds are aliphatic diglycidyl ethers and esters, preferably those derived from a Bisphenol such as Bisphenol A and Bisphenol F and the ethoxylated and propoxylated derivatives thereof, most preferably Bisphenol A diglycidyl ether (BADGE).

Examples of polyglycidyl compounds which may be used include for example Epoxy Novolac resins, triglycidylethers of propoxylated glycerin, or triglycidylethers of castor oil.

As mentioned above, in the reaction of compound B with epoxy compound A to produce compound AB, mono epoxy compounds and in particular mono glycidyl compounds may be used as partial replacement of the polyepoxy compound A, in casu of polyglycidyl compounds A. The mono epoxy compounds have only one free or unreacted epoxy function and may be particularly useful if compound B has more than one moiety reactive toward an epoxy function, in which case the mono epoxy compounds, in casu the monoglycidyl compounds, help to keep the molecular weight of the product low, and hence help in keeping the viscosity of the product formed down. When present, the amount of mono epoxy compounds, in casu monoglycidyl compounds, is preferably limited to representing at most 50%, preferably at most 20% of the total presence of free or unreacted epoxy functions. Suitable examples of mono epoxy compounds are 1-propanol-2,3-epoxyneodecanoate, obtainable as Glydexx N-10 from ExxonMobil Chemical or as Cardura E10P from Hexion Specialty Chemicals, phenyl glycidyl ether, or any long chain hydrocarbon monoglycidyl ether such as stearyl mono glycidyl ether.

In the present invention compound B comprises in the same molecule at least one sulphur-containing anionic moiety and at least one moiety reactive toward an epoxy function, which in casu may be a glycidyl function. Preferably the number of groups reactive toward and epoxy function, in casu glycidyl function, is from 1 to 4, preferably from 2 to 3.

In an embodiment, the compound B used in the production of the compound according to the present invention is selected from carboxylic acids, alcohols, primary and secondary amines, and mixtures thereof, which contain at least one sulphinate or sulphonate anion as part of the molecule, preferably the anion being neutralized into a salt, preferably with a monobasic material, most preferably the sodium salt, most preferably a polyamine sulphonate. Preferably the number of sulphinate and/or sulphonate groups per compound B is from 1 to 3, most preferably this number is 1.

In an embodiment containing the sulphonate anion, the compound B may for example be, or be a derivative of, a material selected from the group consisting of: sulpho-acetic acid, 3-hydroxypropane-1-sulphonic acid and its sodium salt, 3-amino-1-propanesulphonic acid, DL-homocysteic acid, sulphanilic acid and its meta and ortho isomers, 3-amino-4-hydroxybenzenesulphonic acid, 2,4-diaminobenzene sulphonic acid and its isomers, 5-sulphosalicylic acid, 4-aminotoluene-3-sulphonic acid and its isomers, 3-amino-4-methoxybenzenesulphonic acid and its isomers, taurine or 2-aminoethanesulphonic acid, isethionic acid and its sodium and ammonium salt, 4-sulphobenzoic acid and its potassium salt, 3-sulphobenzoic acid and its sodium salt, 4-amino-1-naphthalene sulphonic acid and its sodium salt, N-(2-hydroxy-3-sulphopropyl)-3,5-dimethoxyaniline and its sodium salt, and mixtures thereof. Preferred are 3-amino-4-hydroxybenzenesulphonic acid, taurine or 2-aminoethanesulphonic acid, 4-amino-1-naphthalene sulphonic acid sodium salt and N-(2-Hydroxy-3-sulphopropyl)-3,5-dimethoxyaniline sodium salt.

In an embodiment containing the sulphinate anion, the compound B may for example be, or be a derivative of, a material selected from the group consisting of: hydroxymethane sulphinic acid and its sodium salt, in particular the hydrate, Hypotaurine (C₂H₇NO₂S), and cysteine sulphinic acid and its salts.

In a preferred embodiment of the present invention, the stoichiometric ratio in step (a) of epoxy functions in the epoxy compound A relative to the amount of moieties reactive toward an epoxy function in the compound B, is in the range from 1.5 to 10.0, preferably from 2.0 to 4.0.

Typical but not limiting suitable examples of compound B are: sodium dihydrogen 2-sulphanatosuccinate, such as TEGO® Sodio Sulfosuccinic acid or TEGO® SSSA obtainable from Goldschmidt catalysts, sodium 5-sulpho-isophtalate or monosodium 5-Sulpho-isophthalate (SSIP) such as obtainable from TCI Europe, α,ω-polypropyleneglycol-diamine-sulphopropylated sodium salt (Polyeps 520, Na-salt, obtainable from Raschig GmbH); 1,3-diol-2-ethyl-2-polyethylene-polypropyleneglycol sulphonate salt of sodium, such as Tegomer DS-3135 obtainable from Goldschmidt, now part of EVONIK, also known as polyether-1,3-diol-sulfonate, and 1-propanesulphonic acid-2-hydroxy-3-(2-propenyloxy)-sodium salt (SPAE) obtainable from Raschig GmbH.

Compound B may also be an oligomer or a polymer made with any one of these products named above as suitable compounds B, provided the oligomer or polymer still contains at least one group reactive toward an epoxy function. Not limiting suitable examples are the reaction products of a carboxylic acid functional sulphur-containing compound with any one of polyamines, polyols, lactones or epoxy resins, the reaction products of an amine or alcohol functional sulphur-containing anionic compound with polyacids, lactones, acid anhydrides, polyisocyanates, and epoxy resins. All these reactions are preferably conducted under conditions which limit the occurrence of reactions with the sulphur-containing acid or sulphonate salt itself.

In the context of the present invention, compound C is a compound comprising at least one carboxylic acid moiety and at least one ethylenically unsaturated carbon-carbon double bond. These two moieties may be closely related, such as in a (meth)acrylate function, which designates either an acrylate or a methacrylate function. The compound C which is used in the production of the compound of the present invention typically is selected from the group consisting of acrylic acid, methacrylic acid,oligomers based on (meth)acrylic acid containing at least one free carboxylic acid function and at least one ethylenically unsaturated carbon-carbon double bond, including the reaction products of (meth)acrylic acid and a lactone, and mixtures thereof. Most preferably compound C is acrylic acid.

In a preferred embodiment of the present invention, the stoichiometric ratio of residual free epoxy functions in the product of step (a) relative to the carboxylic acid moieties in compound C is in the range of 1.0 to 2.0.

In the context of the present invention, compound D is a chain extender containing groups capable of reacting with residual free epoxy functions and/or capable of reacting with (meth)acrylic double bonds which may be present in the ABC compound according to the present invention, via a Michael addition reaction to provide chain extension. We prefer to use a compound D which contains at least two N-H bonds, in which the N atoms may be the same or be different. Typically compound D is selected from primary and secondary amines and polyamines, and mixtures thereof, more preferably the amines being aliphatic amines.

In a preferred embodiment of the present invention, chain extender D is selected from the group consisting of an aliphatic primary monoamine, an aliphatic secondary diamine, and mixtures thereof.

Amines as compounds D are preferably selected from primary amines comprising at least one primary amino group (-NH₂) and/or from secondary diamines comprising at least two secondary amino groups (-NH).

The primary amines suitable for the present invention preferably have a weight average molecular weight (Mw) of from 31 to 300 Dalton, more preferably from 45 to 250 Dalton.

Suitable primary amines respond to the formula R₁-NH₂ (I) wherein R₁ represents an alkyl group, which alkyl group is optionally substituted by a hydroxy, an alkoxy, a tertiary amine and/or an aryl group.

The primary amines may for instance be selected from one or more elements of the group consisting of methylamine, ethylamine, n-propylamine, isopropylamine, n-butylamine, iso-butylamine, sec-butylamine, tert-butylamine, 3-methylbutylamine, n-hexylamine, n-octylamine, n-dodecylamine, 2-ethylhexylamine, iso-nonylamine, cyclopentylamine, cyclohexylamine, 2-methylcyclohexylamine, N,N-(di-tert-butyl)-ethyleneamine, benzylamine, 2-(2-aminoethoxy)ethanol, 5-aminopentanol, ethanolamine, 3-amino-1-propanol, iso-propanolamine, 2-amino-2-methyl-1-propanol, 3-(diethylamino)-propylamine, N,N-dimethyl aminoneopentylamine, 2-(diethylamino)ethylamine, 1-methyl-4-(diethylamino)butylamine, 2,2-(di-tert-butylamino)ethylamine, 3-(dimethylamino)propylamine, 2-methoxyethylamine, 2-ethoxyethylamine, 3-methoxypropylamine, 1-methoxyisopropylamine, 3-ethoxypropylamine, 3-isopropoxypropylamine, 3-(2-methoxyethoxy)propylamine, 3-(2-ethylhexyloxy)-propylamine, furfurylamine, and mixtures thereof.

Preferred are primary alkylamines wherein the alkyl group comprises from 1 to 30 carbon atoms, in particular from 1 to 18 carbon atoms, more particularly from 1 to 14 carbon atoms, optionally substituted by one or more hydroxy groups.

The term "alkyl", as used herein, is defined as including saturated monovalent hydrocarbon radicals having straight chain, branched or cyclic moieties, or combinations thereof.

Especially preferred as primary amines are n-propylamine, 2-(2-aminoethoxy)ethanol, 5-aminopentanol, ethanolamine, 3-amino-1-propanol, iso-propanolamine, 2-amino-2-methyl-1-propanol, and mixtures thereof. Particularly preferred are ethanolamine, iso-propanolamine, 2-amino-2-methyl-1-propanol, and mixtures thereof. Most preferred is ethanolamine, especially mono-ethanolamine.

Suitable secondary diamines suitable for use with the present invention respond to formula R₂NH-R₄-NHR₃ (II) wherein R₂ and R₃ represent, each independently, an alkyl, optionally substituted by hydroxy, alkoxy, tertiary amine and/or aryl. R₂ and R₃ may also be linked to each other in order to form a ring. R₄ is preferably an alkylene bivalent radical, which may be straight chain, cyclic or branched, or an arylalkene bivalent radical, and these radicals may be containing up to 20 carbon atoms and may contain from 1 to 8 ether bridges and/or from 1 to 3 tertiary amine bridges. The term "arylalkene", as used herein is meant to designate an alkylene wherein one or more hydrogen groups are replaced by an aryl group.

Preferably, R₄ in formula (II) is chosen from the group of ethylene, 1,2-propylene, trimethylene, hexamethylene, 2,2-dimethylpropylene, 1-methyltrimethylene, 1,2,3-trimethyltetramethylene, 2-methyl-pentamethylene, 2,2,4- (or 2,4,4-)trimethylhexamethylene, meta-xylylene, 3,5,5-trimethylcyclohexyl-1-ene-3-methylene, bis(cyclohexyl-4-ene)-methane, bis(4-methylcyclohexyl-3-ene)methane, cyclohexyl-1,3-ene, cyclo-hexyl-1,4-ene, 1,4-bis(propoxyl-3-ene)butane, N,N-bis(trimethylene)-methyl-amine, 3,6-dioxaoctylene, 3,8-dioxadodecylene, 4,7,10-trioxa-tridecylene, poly(oxytetramethylene), poly(oxypropylene) with 2 to 15 propylene oxide units, poly(oxypropylene-co-oxyethylene) with 2 to 15 propylene oxide and 2 to 15 ethylene oxide units, and 2,2-dimethylpropylene bivalent radicals.

Preferred cyclic secondary diamines are diazo-cyclo pentanes, pentenes, hexanes, hexenes, heptanes and heptenes. Especially preferred secondary amines are 2-methylpiperazine, di-tert-butyl-ethanediamine, and mixtures thereof.

In another embodiment, the compound according to the present invention comprises as part of the molecule at least one further hydrophilic moiety, different from the at least one sulphur-containing anionic moiety already present, and which is selected from the group consisting of anionic, zwitterionic and non-ionic hydrophilic moieties. This further hydrophilic moiety may optionally also contain sulphur.

At least one of the further hydrophilic moieties may preferably be an anionic moiety which is selected from a carboxylic acid or a phosphoric acid moiety and a salt thereof, preferably a sodium, a potassium or an ammonium salt, more preferably a sodium salt. The anionic moiety is preferable because it provides a higher stability over a wider pH range. Also the carboxylic acid moiety is preferred.

At least one of the further hydrophilic moieties may also be a non-ionic moiety which may be an alkoxylated moiety, preferably a moiety which has been ethoxylated and/or propoxylated, more preferably the alkoxylated moiety comprising from 5 to 20 ethoxy and/or propoxy moieties, most preferably from 5 to 20 ethoxy moieties.

We prefer to determine the molecular weight of the compound according to the invention by gel permeation chromatography (GPC) using polystyrene standards. The number average molecular weight Mn and weight average molecular weight Mw are typically measured by dissolving the compounds in tetrahydrofuran (THF) and injecting them at 40°C on a 3xPLgel 5µm Mixed-D LS 300x7.5mm column with MW range 162 to 377400 g/mol, and which has been calibrated with polystyrenes standards.

In an embodiment, the compound according to the present invention has a weight average molecular weight Mw in the range of 1.000 - 30.000 daltons.

In an embodiment, the radiation curable composition according to the present invention is a dispersion of the compound in water, or in an aqueous or other polar medium. We prefer the composition to be a dispersion in water. Because of the improved stability, we prefer that the composition is at least partially neutralised, such as having a pH of at least 6, preferably at least 7, more preferably at least 8 and even more preferably at least 9. The pH of at least 9 is particularly preferred in the case that aminosulphonates are used as compound B. Typically the pH is at most 11, preferably at most 10. Preferred pH ranges are 6 to 11, and more preferred is 8 to 10.

Typically the composition has a total solids content in the range of 30 to 60 %wt, preferably 40 to 50 %wt.

Typically the composition according to the present invention is having a Cone-and-Plate viscosity measured according to DIN EN ISO 3219 at 20°C and using a shear rate of 20 s⁻¹, of at most 5000 mPa.s, preferably in the range of from 50 to 5000 mPa.s, and more preferably at most 1000 mPa.s.

The compositions according to the invention are preferably cured by ultraviolet irradiation, generally in the presence of a photoinitiator. They may, however, also be cured by electron-beam irradiation, which allows the use of compositions free of photoinitiator.

The functional composition according to the present invention, which preferably is selected from a coating, an ink, a varnish or an adhesive composition, may comprise any further ingredient commonly used in the art like at least one compound selected from the group consisting of cross-linkers, pigments, dyes, rheology modifiers, coalescing aids and coalescing solvents, additives and fillers.

Examples of suitable cross-linkers include polyisocyanate cross-linkers well known in the art, such as blocked polyisocyanates or water-dispersible polyisocyanates, such as Desmodur DN (from Bayer); Rhodocoat XWT2104 (from Perstorp). This allows the use of dual cure, in particular a curing by irradiation being followed by a curing by heat.

The present invention also relates to the preparation of the radiation curable compositions of the invention, which typically are radiation curable aqueous compositions like solutions or dispersions in water, in particular they may be dispersions in water.

In one embodiment, the process according to the present invention therefore further comprises the steps of:
(d) combining the compound selected from the product of step (b) and the product formed in step (c) with water or an aqueous medium to form a solution or a dispersion, preferably having a Cone-and-Plate viscosity measured according to DIN EN ISO 3219 at 20°C and a shear rate of 20 s⁻¹ of at most 5000 mPa.s, and also preferably having a solids content in the range of 30 to 60 %wt, preferably 40 to 50 %wt,
(e) optionally neutralizing at least part of the at least one carboxylic acid moiety from compound C with a base, preferably with sodium hydroxide to form the salt, preferably the sodium salt.

Combining the compound according to the present invention with water is preferably performed under high shear mixing conditions. The neutralisation agent may be added during the preparation of the compound in any of steps (a), (b) or (c), but preferably it is added to the water in step (d).

If the product of step (d) is a dispersion, the applicants prefer to produce an oil-in-water dispersion. The Cone-and-Plate viscosity of the dispersion thus prepared, as measured according to DIN EN ISO 3219 at 20°C and using a shear rate of 20 s⁻¹, is preferably in the range of from 50 to 5000 mPa.s, and more preferably at most 1000 mPa.s.

The aqueous dispersions and solutions according to the present invention generally have a total solids content of from about 30 to 60 wt%, preferably from about 35 to 40 wt%; a viscosity measured at 20°C according to DIN EN ISO 3219 at 20°C and using a shear rate of 20 s⁻¹ of at most 5000 mPa, preferably from 20 to 4000 mPa.s, more preferably from 50 to 500 mPa.s, a pH value of 6 to 11, preferably of 8 to 10, an average particle size of about 10 to 600 nm, preferably 10 to 300 nm, more preferably 50 to 150 nm.

The neutralisation step (e) is generally done by adding an organic or inorganic neutralizing agent, or mixtures thereof, to the compound or to the water. Suitable neutralizing agents include volatile organic tertiary amines such as trimethylamine, triethylamine, triisopropylamine, N5N-dimethylcyclohexylamine, N,N-dimethylaniline, N-methylmorpholine, N-ethylmorpholine, N-methylpyrrolidine and N-methylpiperidine and non-volatile inorganic bases comprising monovalent metal cations, preferably alkali metals such as lithium, sodium and potassium and anions such as hydroxides, hydrides, carbonates and bicarbonates that do not remain in the dispersion as such. The total amount of these neutralizing agents required may be calculated according to the total amount of acid groups to be neutralized. Generally a stoichiometric ratio of about 1:1 is used.

In another embodiment of the process which includes forming a dispersion, the process according to the invention further comprises the step of
(f) reacting the compound selected from the compound present in the dispersion from step (d) and the salt obtained from the neutralizing step (e) with a chain extender D to form a chain extended compound.

The applicants have found that the chain extension reaction is much easier to perform on the compound according to the present invention already in the water phase, either dissolved or dispersed, primarily because of the lower viscosity of the compositions involved in the process.

The reaction with the chain extender is typically performed at a temperature in the range from 5 to 90°C, more preferably from 15 to 50°C.

In an embodiment of this process using a chain extender, the chain extender D preferably is selected from the group consisting of primary and secondary amines and polyamines, and combinations thereof, preferably the amines being aliphatic amines, more preferably the chain extender D being selected from the group consisting of an aliphatic primary monoamine and an aliphatic secondary diamine, and mixtures thereof.

The process according to the present invention may further comprise the step of incorporating the oil-in-water dispersion containing the compound selected from the compound from step (d), the salt from the neutralizing step (e), and the chain extended compound from step (f) into a radiation curable aqueous composition.

In another embodiment, the process according to the present invention may further comprise the step of incorporating the oil-in-water dispersion containing the compound selected from the compound from step (d), the salt from the neutralizing step (e), and the chain extended compound from step (f) into a functional composition selected from a coating, an ink, a varnish and an adhesive composition.

The process according to the present invention may further comprise the step of coating an article at least in part with the functional composition.

The process according to the present invention may further comprise the step of exposing the coated part of the article to at least one treatment selected from UV-radiation and electron beam irradiation, to cure the functional composition. In this process, the article is preferably selected from the group consisting of wood, ceramics, concrete and plastics.

In another embodiment of the use according to the present invention, the coated part of the substrate is located outside the factory coating environment, and may be stationary. The curing may then be performed by exposure to radiation from a mobile electromagnetic radiation device.

The applicants have found that compositions according to the present invention show very high reactivity, allowing to operate at higher line speeds or requiring less irradiative energy in the curing step, and hence allows to increase the productivity of the processing equipment.

The applicants have further found that compositions according to the present invention allow to obtain coatings which after radiation curing show an excellent chemical resistance against water, against solvents and against stains, and a superior mechanical resistance against scratch and abrasion. The coatings made therewith also exhibit a good adhesion on porous and on non-porous substrates. The optical properties are also found to be excellent, and offer good transparency and high gloss of the films made according to the present invention.

The coatings obtained from the compositions according to the present invention may be tailored with respect to their desired mechanical properties, i.e. harder or softer, and with respect to their polymer polarity, such as being even more hydrophilic or on the contrary hydrophobic. This variability of the functional compositions according to the present invention allows covering a wide range of different applications, such as coatings for wood, plastic, glass, metal and concrete. The compositions according to the present invention are also found to be suitable for making inks and varnishes, including overprint varnishes.

The present invention is now illustrated with the following examples, without being limited thereto.

In the examples, the following analytical techniques and performance tests were used:
The amount of ethylenically unsaturated groups present is preferably measured by nuclear magnetic resonance spectroscopy and is expressed in milli-equivalents (meq) per g of solid material. A sample of the composition is typically dried for 1 day at room temperature followed by 12 h at 60°C, and subsequently dissolved in deuterated chloroform (CDCl₃). The sample is then submitted to ¹H-NMR analysis in order to measure the molar concentration of ethylenically unsaturated groups, preferably using 1,3,5-bromobenzene as an internal standard, which has the advantage that the 3 protons in the internal standard show up as one peak on the NMR spectrum and which signal is distinct from the signal of any other aromatic hydrogen atoms which may be present in the sample. The comparison between the peak assigned to the protons of the internal standard and the peaks assigned to the ethylenically unsaturated double bonds allows to calculate the molar concentration of ethylenically unsaturated groups according to the formula (A x B) / C, wherein A is the integration of the signal for the protons on the carbon atoms forming the double bonds present in the sample, B is the number of moles of the internal standard which were added to the sample, and C is the integration of the signal for the protons of the internal standard. In the case wherein the double bond is part of a terminal vinyl group such as in an acrylic acid group, the double bond carbon atoms count 3 protons, and the formula may be applied as such. In the case wherein the double bond carbon atoms count less than 3 protons, the formula needs to be adjusted accordingly.

For determining the particle size of particles dispersed in a liquid, we prefer to use a method based on ISO/DIS 13321, and which may be referred to as Quasi-elastic light scattering (QELS) or Photon correlation spectroscopy (PCS). We prefer to use a Manual AutoSizer Lo-C available from Malvern Instruments Ltd. Further relevant information about this method may be found in the article by R.Finsy, in Adv. Colloid Interface Sci. 52, 79 (1994). The method determines average particle size and the width of the size distribution and is applicable to particle sizes ranging from a few nm up to about 1 µm, or up to the onset of sedimentation. The method assumes that the particles are isotropic and spherically shaped. A sample of dispersion at a suitable concentration is illuminated with a monochromatic and coherent laser light beam, and the scattering of the light at a specific angle is recorded by a detector. The detector output is fed to a correlator. The decay of the autocorrelation function of the scattered light intensity is interpreted in terms of particle size and polydispersity index according to the "Cumulants" method. We prefer to use a 4 mW laser diode with 670 nm wavelength and vertical polarisatiion. We prefer to use a 90° scattering angle and we perform the measurement preferably at a temperature of 25.0 ± 0.1 °C. We prefer to use a concentration giving a count rate of the scattered signal in the range of 10-300 kilocounts per second (kcts/s). We prefer to perform at least 3 repeat measurements of at least 60 seconds duration. We prefer to calibrate the method using dispersions of polystyrene latex wit narrow size distribution and average particle diameters of about 100 nm as measured by DLS, which are commercially available as Nanosphere™ from Duke Scientific Corp. For the dispersion, we prefer to use water for aqueous dispersions, and acetone for organic resin particles.

Viscosity of a liquid was measured as the Cone-and-Plate viscosity according to DIN EN ISO 3219: the viscosity is measured with a rotational viscometer at 20°C with a shear rate of 20 s⁻¹. The viscosity value is expressed in milliPascal second (mPa.s).

Reactivity test: a film of 9 µm thick is applied on white non-absorbing paper and exposed to UV radiation, during 10 minutes at a temperature of 50°C, from a 80 W/cm non focalized medium pressure mercury lamp at a defined conveyer speed. The conveyer speed is varied in order to determine the maximum conveyer speed which may still be used to obtain a fully cured film. The fully-cured nature of the film is tested with the fingernail test and the ADR test, as follows: Fingernails test: The fully cured character or nature of the film is assessed by putting some talc on the surface and rubbing with a finger and then with a cotton wad. As long as a mat aspect is observed, the film is considered as not being fully cured and the conveyer speed must be lowered. Acetone double rub (ADR) test: The coating is also submitted to 50 double rubs with a wad of cotton drenched in acetone. A fully cured film is typically not visually affected by this test. The UV-dose, expressed in conveyer speed (m/min) with a fixed lamp power (W/m),) and which is necessary to pass the two tests, is referred to as the reactivity of the coating.

Adhesion test: a film of 60 µm thickness is applied on sanded wood panels and fully cured as described in the reactivity method. A square pattern is engraved in the coating with a cutter. A string of adhesive tape (Tesa 4104) is pressed on the surface. The tape is then pulled off using a calibrated work and/or energy expressed in Joule. Based on the number of squares removed by the tape, a value of adhesion is given ranging from 0 = perfect result, to 5 = worst result. We prefer to repeat this test with a limited number of different work/energy level in order to increase the reliability of the conclusion.

Hamberger(-Höbel) test: a full coating system is applied on sanded wood panels, cured and placed on the Hamberger-Höbel tester, an apparatus $obtainable from Hamberger. The apparatus is equipped with a screw which may be turned in such a way that the force exerted by a coin on the coating can be varied. The force is increased step by step until a scratch of a few centimeters is formed on the coated surface. The higher the force which may be applied before the scratch appears, the better the scratch resistance of the coating. The scratch resistance is expressed in Newton.

Coin test: A sharp edged coin is taken between forefinger and thumb by the operator, it is pressed firmly on the lacquer surface and pulled, exerting constant force, across the film. In the case of a poor adhesion between the film and surface, scratches will appear and there will be a typical stress whitening of the coating. This procedure is the simplest method for checking the adhesion. It however requires skilled personnel to determine the quality of the treated surface. The results are expressed by ranking the samples relative to each other, preferably by giving the number 1 to the best performing coating.

Erichsen hardness test: The Erichsen hardness is a common method for the measurement of the hardness of protective coatings. The estimated or known spring tension on the instrument (Model 318) is set with the help of a slider. Holding the instrument upright and placing the point of the stylus (Bosch, 0.75 mm) on the test substrate, one subsequently draws a 5 to 10 mm long line at a speed of approximately 10 mm/sec. The stylus should produce a scratch which is barely visible with the naked eye. If the spring force is too high, the scratch is too clearly visible; if it is too low, no scratch appears. The coating hardness corresponds to the applied force (measured in Newtons) which leads to the apparition of a first visible scratch of the coating. A higher hardness is expected to provide a better protection against any exposure to scratching conditions during storage and use.

Tack before cure: This is the evaluation of the tack of a completely dried film before exposure to radiation. The results differentiate between 4 levels of tack: very tacky (similar to a PSA), tacky, slightly tacky, tack free (completely dry).

"Anfeuerung": This is a visual evaluation of the wood appearance after coating, in terms of grain contrast, warmth and absence of haze. The result is expressed as a ranking between the samples, assigning 1 to the best performing coating.

Stain resistance test: The method covers the chemical resistance of a coating of 60 µm thick which was wet applied to a non-porous substrate. We prefer to use half white, half black Leneta paper. The coating was dried for 5 minutes at 60°C and then cured under a UV-lamp (Hg) of 80 W/cm at a speed of 5 m/min. The stain resistance is assessed by putting a test substance onto the coating, covering it with a microscope glass and leaving it on for 4 to 16 hours. The test substances used are ammonia (1 and 3%wt solutions in water), ethanol (50%wt in water), acetone, eosine, mustard, coffee and red wine. The stains are then cleaned off by washing with a couple of rubs using a tissue which was saturated with isopropanol. The remaining stains are assessed visually using a 0 to 5 scale, with 0 assigned to a coating on which no stain remained on the coating. A low score (ideally a score of 0) is expected to provide a better protection against any household product spillage.

Solvent or chemical resistance: The solvent resistance is assessed with acetone double rubs (ADR), by pressing a cotton rag saturated with acetone with a backward and forward motion over the coated surface. One double rub is the sum of a backward and forward stroke on the coated surface. The reported number is the number of double rubs required to break through the coating. A high solvent resistance, typically equivalent to needing more than one hundred acetone double rubs, is necessary to ensure a good protection of the coating and the substrate against any household or industrial product spillage. Similar tests are done respectively with cotton drenched in water, isopropanol (IPA) and methyl ethyl ketone (MEK) (also called the MEK double rub test).

In the stain and chemical resistance, we prefer to use four duplicate samples in order to increase the reliability of the result.

Gloss: In the gloss measurement the coated surface is exposed to the unpolarised light beam of a white lamp using an angle of incidence of 60°. The intensity of the reflected light beam is measured with a photo detector and compared to the intensity of the beam directly emitted by the lamp. The result is expressed in % of reflected light intensity relative to the direct beam intensity. This method is based on ISO 2813 and is preferably made using a Tri-Microgloss apparatus.

Pencil hardness is a simple method for determining the comparative scratch resistance and hardness of a coating, and is performed as follows: An operator points with a pencil at a 45° angle onto a coated panel, which is kept in place on a horizontal surface. The test is repeated with pencils of increasing hardness until one or both of the following defects are marked on the coating: a permanent deformation of the paint without cohesive fracture, or a cohesive fracture of the paint. In other words: a visible scratch or a rupture in the surface of the paint. The reported value is the hardness of the hardest pencil which gave no defect on the coating. This method is based on the ISO 15184 method and is preferably performed using the Elcometer 501 Pencil Hardness Tester.

The dry matter content (also known as total solids content) was measured by gravimetric method involving drying the dispersion or solution during 2h at 120 °C.

### EXAMPLE 1 (according to the invention)

A double-wall glass reactor which was equipped with a mechanical stirrer, a thermocouple, a vapour condenser and a dropping funnel was charged with 226 g of Bisphenol diglycidyl ether (Epikote 828 obtained from Hexion Chemicals) and the temperature of the reactor charge was brought to 60°C. Subsequently, 57.42g of α,ω-polypropyleneglycol-diamine-sulphopropylated sodium salt (Polyeps 520 obtained from Raschig) was added slowly over a period of 15 minutes, and reaction was allowed to proceed during 2 hours and kept at 60°C. The resulting product had an epoxy value of 4.38 meq/g.

After this period a blend of Hycoat OA (which is an epoxy acid catalyst), 64.0g of acrylic acid and 0.35g of hydroquinone was added during a period of 15 minutes. The temperature was increased progressively to 110°C over a time period of 2 hours. After keeping the reactor content for 5 hours at 110°C, the remaining epoxy content had been reduced to 0.82 meq/g, and the acid value was 2.2 mg KOH/g. Subsequently the epoxyacrylate product was cooled down to 60°C.

To the cooled product, 412g of water was added slowly in a period of about one hour, and an oil-in-water dispersion was obtained. The oil-in-water dispersion was further mixed during 1 hour and cooled down to 40°C. The pH was raised to within 9-10 by addition of a 0.01 N NaOH solution in water.

The yellowish dispersion "Ex.1" obtained had a 45%wt solids or Non-Volatiles content, and had the following characteristics: a viscosity at 25°C of 56 mPa.s, a pH of 9.9, an average particle size of about 110 nm and a weight average molecular weight Mw of 1520 with a dispersion d of 1.67. The dispersion remained stable for at least 2 weeks at 40°C.

### EXAMPLE 2: Comparative products

A non-ionic epoxyacrylate dispersion "CT 1" was prepared as the first comparative product, as follows:
A double-wall glass reactor equipped with a mechanical stirrer, a thermocouple, a vapour condenser and a dropping funnel was charged with 500 g of Ebecryl 600 (Bisphenol A diglycidyl ether (BADGE) diacrylate obtained from Cytec) and 100g Viacryl510 (a proprietary non-ionic epoxy emulsifier from Cytec). The two components were mixed at a temperature of about 60°C during a half hour.

Subsequently 400g of water was added slowly during a period of 2 hours under high shear mixing conditions, and the temperature was progressively decreased to 40°C. The dispersion which was obtained was further mixed during a further 3 hours, after the addition of all the water.

Obtained was a yellowish dispersion "CT 1" having a 55%wt total solids content or Non-Volatiles (NV) content, and which had the following characteristics: a viscosity at 20°C of 580 mPa.s, a pH of 6.7, and an average particle size of 550 nm.

As further comparative dispersions, named "CT 2" and "CT 3", were used respectively the Ucecoat 7830 and the Ucecoat 7773, two commercial radiation curable polyurethane dispersions (UV PUD) from Cytec Surface Specialties, and which both are designed for coating wood and for coating plastic.

The dispersion labelled "Ex.1" was tested in a coating on a variety of wood substrates, and on metal, more precisely on Hot Dip Galvanized steel, and each time compared with comparative example "CT 1". In the wood coating test, these were tested also against commercial sample "CT 2". In the metal coating test, they were compared with commercial sample "CT 3". The formulations in which the products were introduced, and the results of the performance tests of the respective coatings, are shown in the tables. Table 1 shows the results on wood. Where more than one coating layer was applied, the previous layer was first sanded before the next coating layer was applied. Table 2 shows the results on the metal substrate.

**Table 1**

| **Formulation** | | **Ex.1** | **CT 1** | **CT 2** |
|---|---|---|---|---|
| Dispersion | | 100 | 82 | 100 |
| water | | - | 18 | - |
| TS 100 | | 1.5 | 1.5 | 1.5 |
| Aquamat 208 | | 3 | 3 | 3 |
| Additol BCPK | | 1.5 | 1.5 | 1.5 |
| Ucecoat 8460 1/1 water solution | | 1 | 1.5 | 1.5 |
| **Performance tests** | **Substrate** | | | |
| Coat weight on wood | 2 Coats on Sapelli | 60 gr/m² | 60 gr/m² | 60 gr/m² |
| | 3 Coats on Beech | 60 gr/m² | 60 gr/m² | 60 gr/m² |
| Tack before cure | | Very Tacky | Very Tacky | Very Tacky |
| Gloss | Sapelli | 50 - 38 | 60 - 42 | 25 - 20 |
| | Beech | 83 - 79 | 85 - 79 | 31 - 27 |
| Anfeuerung * | Sapelli | 1 | 1 | 2 |
| Adhesion ** | Beech | 0 | 0 | 0 |
| | Sapelli | 0 | 0 | 0 |
| Coin test * | Beech | 2 | 3 | 1 |
| Erichsen (N) | Beech | < 6 | < 6 | 9 |
| Hamberger (N) | Beech | 30 | 20 | 30 |
| Resistance ** | On Beech | | | |
| Chemical | NH₃ 1% | 0-0-0-0 | 0-0-0-0 | 0-0-0-0 |
| Chemical | NH₃ 10% | 0-0-0-0 | 0-0-0-0 | 1-1-1-1 |
| Stain | Red Wine | 0-0-0-0 | 0-0-0-0 | 0-0-0-0 |
| Stain | Mustard | 0-0-0-0 | 0-0-1-1 | 0-3-3-3 |
| Chemical | Ethanol 50% | 0-0-0-0 | 0-0-0-0 | 0-0-0-0 |
| Chemical | Acetone | 0-0-0-0 | 0-0-0-0 | 0-0-0-0 |
| Stain | Coffee | 0-0-0-0 | 0-0-0-0 | 0-0-0-0 |
| Stain | Eosine | 0-0-0-0 | 1-1-1-1/2 | 2/3-2/3-3-3 |
| Dubbel Rubs | Water | > 100 | > 100 | > 100 |
| | Acetone | > 100 | > 100 | > 100 |
| | IPA | > 100 | > 100 | > 100 |

| | | | | |
|---|---|---|---|---|
| * ranking from 1 for the best performing coating sample, to 3 for the worst performing coating ** score from 0 for a perfect result, to 5 for a very bad result | | | | |

In the formulations the following additional ingredients were used in the amounts shown in the table:

| | |
|---|---|
| TS 100: | silica from Degussa |
| Aquamat 208: | water-based wax from Degussa |
| Additol BCPK 100: | photoinitiator from Cytec |
| Ucecoat 8460 , 1/1 water sol: | thickener from Cytec |

The results of the tests show that the two water based epoxyacrylate coatings "Ex.1" and "CT 1" are giving the better "anfeuerung" and a higher gloss than the UV PUD comparative example.

The sample "Ex.1" according to the present invention gives the better stain resistance.

**Table 2 coating on metal (Hot Dip Galvanized steel or "HDG")**

| **Formulation** | | **Ex.1** | **CT 1** | **CT 3** |
|---|---|---|---|---|
| Dispersion | | *100* | *100* | *100* |
| Additol BCPK | | *1.5* | *2.2* | *1.5* |
| Ucecoat 8460 1/1 water sol | | *2* | *1* | *1.5* |
| **Test results** | | | | |
| Reactivity | *10' at 50°C* - *80W*/*cm -thickness 9 µm* | | | |
| | *Maximum speed which gives 100% cure* | | | |
| Fingernails | m/min | 35 | 40 | 15 |
| ADR | m/min | >50 | >50 | 30 |
| Coating on HDG | *10' at 50°C - 2x 10m*/*min - 80W*/*cm -* | | | |
| *thickness* | *µm* | 12 | 14 | 12 |
| Gloss | | 90 | 102 | 54 |
| MEK double rub | | >100 | >100 | >100 |
| Pencil hardness | | 6H | 2H | 2H |
| Adhesion ** | | | | |
| 18J | | 1 | 4 | 1 |
| 14.75 J | | 1 | 2 | 1 |
| 2J** | | 1 | 1 | 1 |
| Corrosion resistance | Ranking after 240h | | | |
| | Ex.1 and CT 1 >> CT 3 | | | |

| | | | | |
|---|---|---|---|---|
| * ranking from 1 for the best performing coating to 3 for the worst. ** score from 0 for a perfect result to 5 for a very bad result. | | | | |

The results in Table 2 show that the two water based epoxyacrylates ("Ex.1" and CT 1") have a much higher reactivity than the comparative example UV PUD "CT 3".

It may also be noticed that "Ex. 1" combines an excellent adhesion to HDG (Hot Dip Galvanized steel), which was found to be very similar to what was obtained with the anionic UV PUD, with an excellent salt spray resistance as was obtained with the non-ionic water based epoxyacrylate "CT 1 ".

Also the corrosion resistance of the two water based samples Ex.1 and CT 1 were better than what was obtained with the UV PUD "CT 3".

Having now fully described this invention, it will be appreciated by those skilled in the art that the invention can be performed within a wide range of parameters within what is claimed, without departing from the spirit and scope of the invention. As understood by those of skill in the art, the overall invention, as defined by the claims, encompasses other preferred embodiments not specifically enumerated herein.

## Claims

1. A compound comprising at least one epoxy function and at least one ethylenically unsaturated function, whereby the epoxy functions present in the compound are independently selected from the group consisting of unreacted epoxy functions and epoxy functions which have been reacted with a moiety reactive toward an epoxy function, the compound further comprising at least one sulphur-containing anionic moiety.

2. The compound according to claim 1 comprising a plurality of the epoxy functions, preferably at least one of the epoxy functions being a glycidyl function.

3. The compound according to claim 1 or 2 which is an epoxy (meth)acrylate.

4. The compound according to any one of the preceding claims obtained from
(a) in a first step, producing a compound AB by reacting at least one compound B comprising in the same molecule at least one sulphur-containing anionic moiety and at least one moiety reactive toward an epoxy function, with at least one epoxy compound A, which comprises at least two unreacted epoxy functions, and, if compound B comprises per molecule at least two moieties reactive towards an epoxy function, optionally in the presence of at least one mono epoxy compound, whereby the unreacted epoxy functions are present in a stoichiometric excess relative to the number of moieties reactive toward an epoxy function, and
(b) in a second step, producing a compound ABC by reacting at least partially and preferably completely the residual free or unreacted epoxy functions in the product AB of step (a) with a compound C comprising at least one carboxylic acid moiety and at least one ethylenically unsaturated carbon-carbon double bond,
(c) optionally, in a further step producing a chain extended compound ABCD by reacting the product ABC of step (b) with at least one chain extender D containing groups capable of reacting with residual free epoxy functions and/or capable of reacting with (meth)acrylic double bonds via a Michael addition reaction to provide chain extension.

5. The compound according to any one of the preceding claims in which the sulphur-containing anionic moiety is selected from a sulphinic acid moiety, a sulphonic acid moiety, the salts thereof, and mixtures thereof.

6. The compound according to any one of the preceding claims wherein the sulphur-containing anionic moiety is a sulphonic acid moiety, preferably a salt thereof.

7. The compound according to any one of the preceding claims which is soluble in an aqueous medium.

8. The compound according to any one of the preceding claims which is dispersible in an aqueous medium.

9. A radiation curable composition comprising at least one compound according to any one of the preceding claims.

10. The radiation curable composition according to claim 9 which is selected from a solution of the compound in an aqueous medium and a dispersion of the compound in an aqueous medium, preferably the dispersion.

11. A functional composition selected from a coating, an ink, a varnish or an adhesive composition, comprising at least one compound according to any one of claims 1-8 or comprising the radiation curable composition according to any one of claims 9-10.

12. An article which at least for a part is covered by a functional composition according to claim 11, the article preferably comprising at least a part made of a material selected from wood, paper, plastic, glass, metal, ceramics and concrete, more preferably the article being selected from the group consisting of a tile, a wooden floor element, parquet, parquetry and furniture.

13. A process for the production of the compound according to any one of claims 1 to 8, comprising the steps of
(a) in a first step, producing a compound AB by reacting at least one compound B comprising in the same molecule at least one sulphur-containing anionic moiety and at least one moiety reactive toward an epoxy function, with at least one epoxy compound A, which comprises at least two unreacted epoxy functions, and, if compound B comprises per molecule at least two moieties reactive towards an epoxy function, optionally in the presence of at least one mono epoxy compound, whereby the unreacted epoxy functions are present in a stoichiometric excess relative to the number of moieties reactive toward an epoxy function, and
(b) in a second step, producing a compound ABC by reacting at least partially and preferably completely the residual free or unreacted epoxy functions in the product AB of step (a) with a compound C comprising at least one carboxylic acid moiety and at least one ethylenically unsaturated carbon-carbon double bond,
(c) optionally, in a further step producing a chain extended compound ABCD by reacting the product ABC of step (b) with at least one chain extender D containing groups capable of reacting with residual free epoxy functions and/or capable of reacting with (meth)acrylic double bonds via a Michael addition reaction to provide chain extension.

14. Use of the compound according to any one of claims 1-8 for the at least partial covering of a substrate selected from the group consisting of wood, paper, plastic, glass, metal, ceramics and concrete.

15. Use of the compound according to any one of claims 1-8 in a water-based radiation curable printing ink.
